(19) Europäisches Patentamt
European Patent Office
Office européen des brevets

(11) Veröffentlichungsnummer: **0 063 099**
**B1**

(12) # EUROPÄISCHE PATENTSCHRIFT

(45) Veröffentlichungstag der Patentschrift: **27.11.85**

(51) Int. Cl.⁴: **C 07 D 249/08**, A 01 N 43/50, C 07 F 9/65

(21) Anmeldenummer: **82810098.2**

(22) Anmeldetag: **04.03.82**

(54) **Verfahren zur Herstellung von Phenyläthyltriazolen.**

(30) Priorität: **10.03.81 CH 1623/81**

(43) Veröffentlichungstag der Anmeldung: **20.10.82 Patentblatt 82/42**

(45) Bekanntmachung des Hinweises auf die Patenterteilung: **27.11.85 Patentblatt 85/48**

(84) Benannte Vertragsstaaten: **AT BE CH DE FR GB IT LI NL**

(56) Entgegenhaltungen:
**EP - A - 0 008 651**
**EP - A - 0 060 223**
**DE - A - 2 735 872**

(73) Patentinhaber: **CIBA-GEIGY AG, Postfach, CH-4002 Basel (CH)**

(72) Erfinder: **Maier, Ludwig, Dr., Im Lee 28, CH-4144 Arlesheim (CH)**
Erfinder: **Kunz, Walter, Dr., Im Goldbrunnen 55, CH-4104 Oberwil (CH)**

**EP 0 063 099 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft ein neues Verfahren zur Herstellung von 1-(2-Phenyläthyl)-1H-1,2,4-triazolen.

Ferner betrifft die Erfindung neue 1H-1,2,4-Triazol -1- yl-methylphosphonate und 1H-1,2,4-Triazol -1- yl-methylphosphoniumsalze, die als Ausgangsmaterialien zur Durchführung des erfindungsgemässen Verfahrens dienen sowie Verfahren zur Herstellung dieser Ausgangsprodukte.

Die nach dem neuen erfindungsgemässen Verfahren herstellbaren 1-(2-Phenyläthyl) -1H-1,2,4-triazole sind als Fungizide aus der deutschen Offenlegungsschrift 2 735 872 bekannt.

Da einerseits diese Verbindungen sich als hochaktive Fungizide mit einem günstigen Fungizidspektrum erwiesen haben, andererseits das beschriebene Syntheseverfahren im Bezug auf die Ausbeuten und die industrielle Anwendbarkeit keine befriedigende Lösung darstellte, bestand das Bedürfnis, ein neues Herstellungsverfahren für die genannten 1-(2-Phenyläthyl) -1H-1,2,4-triazole bereitzustellen, das zugleich höhere Ausbeuten liefert und eine einfache Reaktionsführung erlaubt.

Die nach dem neuen erfindungsgemässen Verfahren herstellbaren 1-(2-Phenyläthyl) -1H-1,2,4-triazole entsprechen der allgemeinen Formel I,

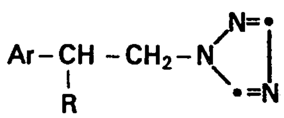
(I),

worin

Ar einen gegebenenfalls durch $C_1$–$C_4$-Alkyl, $C_1$–$C_4$-Alkoxy, Cyan, Nitro, Trifluormethyl oder ein bis drei Halogenatome substituierten Phenylrest bedeutet und

R für Wasserstoff; $C_1$–$C_{10}$-Alkyl; $C_2$–$C_5$-Alkenyl; $C_3$–$C_8$-Cycloalkyl; gegebenenfalls durch $C_3$–$C_8$-Cycloalkyl oder Phenyl substituiertes $C_1$–$C_4$-Alkyl steht, wobei der Phenylsubstituent seinerseits gegebenenfalls durch Halogen, $C_1$–$C_4$-Alkyl oder $C_1$–$C_4$-Alkoxy substituiert sein kann.

Unter Alkyl sind je nach der Anzahl der Kohlenstoffatome zu verstehen: Methyl, Äthyl, n-Propyl oder 1-Propyl, sowie die isomeren Reste von Butyl, Pentyl, Hexyl, Heptyl, Octyl, Nonyl oder Decyl.

Beispiele für Alkenyl sind: Vinyl, Allyl, 1-Propenyl, 1-Methyl-2-propenyl, 2-Methyl-2-propenyl, 2-Butenyl oder 3-Butenyl, sowie isomere Pentenylreste.

Unter Alkoxy werden verstanden: Methoxy, Äthoxy, n-Propyloxy oder i-Propyloxy sowie isomere Butyloxy.

Definitionsgemässe Cycloalkylreste sind die Reste von Cyclopropan, Cyclobutan, Cyclopentan, Cyclohexan, Cycloheptan oder Cyclooctan.

Halogen bedeutet in der Regel Fluor, Chlor, Brom oder Jod, vorzugsweise aber Fluor, Chlor und Brom.

Beispiele für Phenylalkyl sind Benzyl, 2-Phenyläthyl, 3-Phenylpropyl, 2-Phenylpropyl, insbesondere aber Benzyl.

Gemäss vorliegender Erfindung wird vorgeschlagen, die 1-(2-Phenyläthyl) -1H-1,2,4-triazole der allgemeinen Formel I in der Weise herzustellen, dass man ein Phenylketon der Formel II,

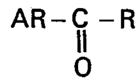
(II),

worin Ar und R die unter Formel I angegebene Bedeutung haben, zunächst durch Umsetzung mit einem 1H-1,2,4-Triazol -1-yl-methylphosphonat der Formel III

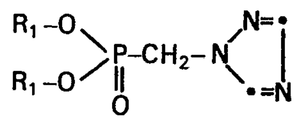
(III),

worin die Reste $R_1$ unabhängig voneinander Phenyl oder $C_1$–$C_4$-Alkyl bedeuten, oder mit einem Phosphoniumsalz der Formel IV,

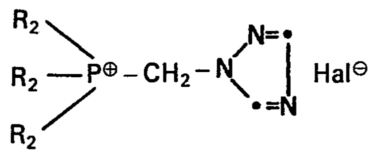
(IV),

worin die Reste $R_2$ unabhängig voneinander Phenyl oder gegebenenfalls durch Hydroxyl substituiertes $C_1$–$C_4$-Alkyl bedeuten, und Hal für Chlor, Brom oder Jod steht, in einem inerten organischen Lösungsmittel in Gegenwart einer starken Base zu einem 2-(1H-1,2,4-Triazol-1-yl)-styrol der Formel V,

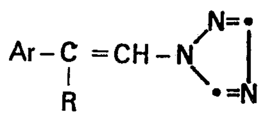
(V),

worin Ar und R die unter Formel I angegebenen Bedeutungen haben, umsetzt und dieses anschliessend durch katalytische Hydrierung in die Verbindung der Formel I überführt.

Die Überführung der Phenylketone der Formel II in die Triazolylstyrole der Formel V kann nach dem erfindungsgemässen Verfahren erfolgen entweder unter Verwendung von Phosphonaten der Formel III oder von Phosphoniumsalzen der Formel IV.

Die Reaktionsvariante, die als Reaktionspartner die Phosphonate der Formel III verwendet, wird vorteilhafterweise so geführt, dass man das Phosphonat zunächst mit einer starken Base und danach mit dem Phenylketon der Formel II versetzt oder dass man eine Lösung des Gemisches von Phosphonat und Phenylketon zu einer Lösung oder einer Suspension einer starken Base zusetzt.

Es hat sich dabei als günstig erwiesen, wenn man die Base im Überschuss zusetzt. Üblicherweise beträgt der Überschuss weniger als das Doppelte der zur Reaktion benötigten molaren Menge, also zwischen 0,01 und 1 Äquivalenten.

Als Lösungsmittel bei dieser Eintopfreaktion können inerte, polare, aprotische Lösungsmittel verwendet werden. Solche Lösungsmittel sind Äther wie Diäthyläther, Tetrahydrofuran, Dioxan, Dimethoxyäthan oder Diäthylenglykol-dimethyläther; Säureamide wie Dimethylformamid, 2-Pyrrolidinon oder Hexamethylphosphorsäuretriamid; und Sulfoxide wie Dimethylsulfoxid.

Bevorzugt sind Lösungsmittel, deren Siedepunkt über 60 °C liegt, also z.B. Tetrahydrofuran, Dioxan, Dimethoxyäthan, Dimethylformamid oder Dimethylsulfoxid.

Als starke Basen können verwendet werden: metallorganische Verbindungen wie Methyllithium, Propyllithium, Butyllithium, Phenyllithium oder Triphenylmethylnatrium; Alkoholate wie Natriummethylat, Natriumäthylat, Kaliumäthylat oder Kalium-tert.-butylat; Metallhydride wie Lithiumhydrid, Natriumhydrid oder Calciumhydrid; und Alkaliamide wie Natriumamid oder Lithiumdiisopropylamid.

Als bevorzugte Basen sind die Metallhydride, die metallorganischen Verbindungen und die Alkoholate zu verstehen.

Da es sich sowohl bei der Umsetzung des Phosphonats III mit der starken Base zum entsprechenden Salz, als auch bei der weiteren Reaktion mit dem Keton II um exotherme Vorgänge handelt, kühlt man das Reaktionsgefäss jeweils vor dem Zusetzen des Reagenzes ab und erwärmt dann anschliessend zur Vervollständigung der Umsetzung. Geeignete Temperaturintervalle sind für die erste Stufe −40° bis +40 °C, insbesondere −20° bis +40 °C und für die zweite Stufe −20° bis +80 °C, insbesondere 0° bis +60 °C.

Das entstandene Triazolylstyrol der Formel V wird isoliert, indem man das Reaktionsgemisch mit Wasser versetzt, einem mit Wasser nicht mischbaren Lösungsmittel extrahiert und die organischen Phasen eindampft.

Die Reaktionsvariante, die als Reaktionspartner die Phosphoniumsalze der Formel IV verwendet, wird vorteilhafterweise so geführt, dass man das Phosphoniumsalz zunächst mit einer starken Base und danach mit dem Phenylketon der Formel II versetzt.

Es hat sich dabei als günstig erwiesen, wenn man die Base im Überschuss zusetzt. Üblicherweise beträgt der Überschuss weniger als das Doppelte der zur Reaktion benötigten molaren Menge, also zwischen 0,01 und 1 Äquivalenten.

Als Lösungsmittel bei dieser Eintopfreaktion können inerte, polare, aprotische Lösungsmittel verwendet werden. Solche Lösungsmittel sind Äther wie Diäthyläther, Tetrahydrofuran, Dioxan, Dimethoxyäthan oder Diäthylenglykol-dimethyläther; Säureamide wie Dimethylformamid, 2-Pyrrolidinon oder Hexamethylphosphorsäuretriamid; und Sulfoxide wie Dimethylsulfoxid.

Bevorzugt sind Lösungsmittel, deren Siedepunkt über 80 °C liegt, also z.B. Dioxan, Dimethoxyäthan, Diäthylenglykol-dimethyläther, Dimethylformamid, 2-Pyrrolidinon oder Dimethylsulfoxid.

Als starke Basen können verwendet werden: metallorganische Verbindungen wie Methyllithium, Propyllithium, Butyllithium, Phenyllithium, oder Triphenylmethylnatrium; Alkoholate wie Natriummethylat, Natriumäthylat, Kaliumäthylat oder Kalium-tert.-butylat; Metallhydride wie Lithiumhydrid, Natriumhydrid oder Calciumhydrid; und Alkaliamide wie Natriumamid oder Lithiumdiisopropylamid.

Als bevorzugte Basen sind die Metallhydride und die metallorganischen Verbindungen zu verstehen.

Da es sich sowohl bei der Umsetzung des Phosphoniumsalzes mit der starken Base zum entsprechenden Ylid, als auch bei der weiteren Reaktion mit dem Keton II um exotherme Vorgänge handelt, kühlt man das Reaktionsgefäss jeweils vor dem Zusetzen des Reagenzes ab und erwärmt dann anschliessend zur Vervollständigung der Umsetzung. Geeignete Temperaturintervalle sind für die erste Stufe −40° bis +80 °C, insbesondere −20° bis +40 °C und für die zweite Stufe −20° bis +110 °C, insbesondere 0° bis +60 °C.

Das entstandene Triazolylstyrol der Formel V wird isoliert, indem man das Reaktionsgemisch mit Diäthyläther verdünnt, das ausgefallene Phosphinoxid abfiltriert und das Filtrat eindampft. Um am Phosphinoxid anhaftendes Produkt zu gewinnen, kann es notwendig sein, den Filterkuchen mehrmals mit Äther auszuwaschen.

Bei beiden Varianten in denen man als Reaktionspartner Phosphorverbindungen der Formeln III oder IV verwendet, kann sich der Zusatz eines Kronenäthers als sehr vorteilhaft erweisen. So ist beispielsweise bei der Verwendung von Kaliumhaltigen Basen und/oder Natrium-haltigen Basen zur Erzeugung der Anionen der Einsatz von [18]-crown-6 oder von [15]-crown-5 sehr günstig.

Bei diesen Herstellungsschritten entstehen üblicherweise Gemische von cis- und trans-Olefinen, in denen der cis-Olefinanteil im allgemeinen überwiegt. Die Bildung eines höheren trans-Olefinanteils kann durch einen grösseren Überschuss am Ylid oder durch Zusatz von Lithiumsalzen (z.B. $LiClO_4$) erzielt werden. [Vgl. M. Schlosser, Chem. Ber. 103, 2841].

Die katalytische Hydrierung innerhalb des erfindungsgemässen Verfahrens der Triazolylstyrole der Formel V zu Phenyläthyltriazolen der Formel I erfolgt im allgemeinen mit elementarem Wasserstoff an einem Edelmetallkatalysator.

Als geeignete Katalysatoren haben sich Palladium/Bariumsulfat, Palladium/Kohle, Rhodium/Kohle, Platinoxid, Rhodiumoxid, Platinmoor, Rhodiummoor, Raney-Nickel oder auch Komplexe wie der Rhodiumtriphenylphosphinkomplex erwiesen.

Von den genannten Edelmetallkatalysatoren können bevorzugt die Metalloxide, wie Platinoxid und Rhodiumoxid, verwendet werden.

Ausserdem hat sich herausgestellt, dass sich besonders gut zur Hydrierung der Triazolylstyrole

der Formel V Gemische von Edelmetallkatalysatoren eignen.

Ein solches besonders günstiges Katalysatorgemisch ist ein Gemisch aus Rhodiumoxid und Platinoxid.

Üblicherweise werden die Hydrierungen der Triazolylstyrole der Formel V in einem inerten organischen Lösungsmittel durchgeführt. Solche Lösungsmittel sind Ester wie Äthylacetat; Alkohole wie Methanol, Äthanol, n-Propanol oder i-Propanol; Äther wie Diäthyläther, Tetrahydrofuran, Dioxan oder Dimethoxyäthan; Kohlenwasserstoffe wie Pentan, Hexan, Cyclohexan, Benzol, Toluol oder Xylol; und Säuren wie Essigsäure, Trifluoressigsäure.

Bevorzugt wird die Hydrierung in Säuren wie Essigsäure und Trifluoressigsäure ausgeführt. Jedoch kann das saure Medium auch durch Zusatz von Mineralsäuren wie Schwefelsäure, Phosphorsäure oder Salzsäure zu den aufgeführten inerten Lösungsmitteln hergestellt werden.

Die Hydrierung wird bei Temperaturen zwischen 0° und +100 °C, vorzugsweise zwischen 10° und 30 °C, und bei Drücken von 1 bis 100 Bar, vorzugsweise von 1 bis 8 Bar, ausgeführt.

Die Endprodukte der Formel I dieses Herstellungsverfahrens werden isoliert, indem man den Katalysator abtrennt, die verbleibende Lösung einengt und den Rückstand durch Destillation, Kristallisation oder Chromatographie reinigt.

Die Verbindungen der Formel I besitzen für den Fall R $\neq$ Wasserstoff im Kohlenstoffatom 2 ein Asymmetriezentrum. Da diese Asymmetrie im Verlaufe einer nicht-stereospezifischen Reaktion (Hydrierung) entsteht, fallen die Verbindungen meist als Racemate an und werden als solche verwendet. Dabei ist es unwesentlich, ob die E- oder Z-Form oder das E–Z-Isomerengemisch des entsprechenden Olefins hydriert wird. Für den Fall, dass im Rest R weitere Asymmetriezentren vorliegen, entstehen Diastereomeren-Gemische von Verbindungen der Formel I, die nach bekannten Methoden in Enantiomere getrennt werden können.

Eine bevorzugte Verfahrensvariante unter Verwendung des Phosphonats der Formel III ist ein Verfahren, in dem man bei der Umsetzung des Phenylketons der Formel II mit dem Methylphosphonat der Formel III zunächst das Phosphonat mit einem Überschuss (bis zur doppelten molaren Menge) einer Base, ausgewählt aus der Gruppe metallorganische Verbindungen, Alkoholate, Metallhydride ode Alkaliamide, in einem inerten, polaren, aprotischen Lösungsmittel bei einer Temperatur von –40° bis +40°C und dann mit dem Keton der Formel II bei Temperaturen zwischen –20° und +80°C zum Triazolylstyrol der Formel V umsetzt und dieses anschliessend mit einem Edelmetallkatalysator in neutralem oder saurem Medium bei Temperaturen von 0° bis 100°C und Drücken von 1 bis 100 Bar hydriert.

Eine weitere Bevorzugung liegt darin, dass man bei diesem Verfahren für die Umsetzung des Phosphonats als Base ein Metallhydrid oder eine metallorganische Verbindung, als Lösungsmittel Äther, Säureamide oder Sulfoxide verwendet und die Temperatur zwischen –20 und +40°C wählt; die Umsetzung mit dem Keton bei Temperaturen zwischen 0° und 60°C ausführt und das Triazolylstyrol mit einem Rhodiumoxid/Platinoxid-Gemisch bei 10° bis 30°C und 1 bis 8 Bar hydriert.

Eine bevorzugte Verfahrensvariante unter Verwendung des Phosphoniumsalzes der Formel IV ist ein Verfahren, in dem man bei der Umsetzung des Phenylketons der Formel II mit dem Methylphosphoniumsalz der Formel IV zunächst das Phosphoniumsalz mit einem Überschuss (bis zur doppelten molaren Menge) einer Base, ausgewählt aus der Gruppe metallorganische Verbindungen, Alkoholate, Metallhydride oder Alkaliamide, in einem inerten, polaren, aprotischen Lösungsmittel bei einer Temperatur von –40° bis +80 °C und dann mit dem Keton der Formel II bei Temperaturen zwischen –20° und +110 °C zum Triazolylstyrol der Formel V umsetzt und dieses anschliessend mit einem Edelmetallkatalysator in neutralem oder saurem Medium bei Temperaturen von 0 ° bis 100 °C und Drücken von 1 bis 100 Bar hydriert.

Eine weitere Bevorzugung liegt darin, dass man bei diesem Verfahren für die Umsetzung des Phosphonats als Base ein Metallhydrid oder eine metallorganische Verbindung, als Lösungsmittel Äther, Säureamide oder Sulfoxide verwendet und die Temperatur zwischen –20° und +40 °C wählt; die Umsetzung mit dem Keton bei Temperaturen zwischen 0° und 60 °C ausführt und das Triazolylstyrol mit einem Rhodiumoxid/Platinoxid-Gemisch bei 10° bis 30 °C und 1 bis 8 Bar hydriert.

Die Ausgangsverbindungen der Formel II sind zum Teil bekannt und im Handel erhältlich oder sie können durch Friedel-Crafts-Acylierung aus entsprechenden Carbonsäurehalogeniden und Benzolen leicht hergestellt werden.

Die Phosphonate der Formel III und die Phosphoniumsalze der Formel IV sind neu und bilden daher zusammen mit den Verfahren zu ihrer Herstellung einen Aspekt der Erfindung.

Die 1H-1,2,4-Triazol-1-yl-methylphosphonate der allgemeinen Formel III,

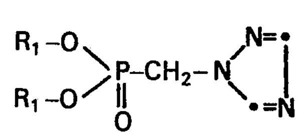

(III),

worin die Reste $R_1$ unabhängig voneinander Phenyl oder $C_1$–$C_4$-Alkyl bedeuten, werden erfindungsgemäss hergestellt, indem man ein 1-Halogenmethyl-1H-1,2,4-triazol der Formel VI,

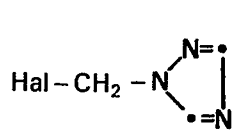

(VI),

worin Hal für Chlor, Brom oder Jod steht, entweder mit einem sekundären Phosphit der Formel VII,

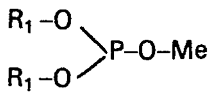 (VII),

worin $R_1$ die unter Formel III gegebene Bedeutung hat und Me für ein Alkalimetallatom steht, oder mit einem tertiären Phosphit der Formel VIII umsetzt,

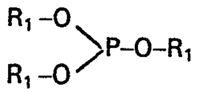 (VIII),

worin $R_1$ die unter Formel III gegebene Bedeutung hat.

Mit Vorteil wird das Verfahren zur Herstellung der Phosphonate der Formel III in einem inerten organischen Lösungsmittel durchgeführt. Solche Lösungsmittel sind aromatische Kohlenwasserstoffe wie Benzol, Toluol oder Xylol; Äther wie Diäthyläther, Tetrahydrofuran, Dioxan, Dimethoxyäthan oder Diäthylenglykol-dimethyläther oder Nitrile wie Acetonitril. Verwendet man ein sekundäres Alkaliphosphit der Formel VII, ist es angebracht ein polares Lösungsmittel wie Acetonitril, Dimethoxyäthan oder Diäthylenglykol-dimethyläther einzusetzen. Wird zur Synthese der Phosphonate ein tertiäres Phosphit der Formel VIII eingesetzt, so kann dieses selbst häufig als Lösungsmittel dienen.

In jedem Fall jedoch empfiehlt es sich, die Reaktionsmischung zu erwärmen: bei der sekundär-Phosphitreaktion auf 50° bis 150 °C, vorzugsweise auf 80° bis 120 °C und bei der tertiär-Phosphitreaktion auf 100° bis 180 °C, vorzugsweise auf 120° bis 160 °C.

Bei den sekundären Alkaliphosphiten handelt es sich im allgemeinen um Natrium- oder Kaliumphosphite.

Die Phosphite der Formel III werden isoliert, indem man gegebenenfalls den entstandenen Niederschlag abtrennt, die Lösung eindampft und den Rückstand destilliert.

Die 1H-1,2,4-Triazol-1-yl-methylphosphoniumsalze der allgemeinen Formel IV,

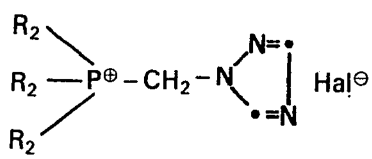 (IV),

worin die Reste $R_2$ unabhängig voneinander Phenyl oder gegebenenfalls durch Hydroxyl substituiertes $C_1$–$C_4$-Alkyl und Hal Chlor, Brom oder Jod bedeuten, werden erfindungsgemäss hergestellt, indem man ein 1-Halogenmethyl-1H-1,2,4-triazol der Formel VI mit einem Phosphin der Formel IX umsetzt,

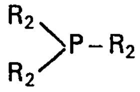 (IX),

worin $R_2$ die unter Formel IV gegebene Bedeutung hat.

Mit Vorteil wird das Verfahren zur Herstellung der Phosphoniumsalze der Formel IV in einem inerten, organischen Lösungsmittel durchgeführt. Solche Lösungsmittel sind aromatische Kohlenwasserstoffe wie Benzol, Toluol oder Xylol; Äther wie Diäthyläther, Tetrahydrofuran, Dioxan, Dimethoxyäthan oder Diäthylenglykol-dimethyläther; Säureamide wie Dimethylformamid oder 2-Pyrrolidinon oder Nitrile wie Acetonitril. Vorzugsweise werden polare Lösungsmittel wie Dimethylformamid, Acetonitril oder Dimethoxyäthan verwendet.

Es empfiehlt sich, das Reaktionsgemisch auf 30° bis 120 °C, vorzugsweise auf 50° bis 100 °C, zu erwärmen.

Beim Abkühlen der Reaktionsmischungen kristallisieren die Phosphoniumsalze meist in reiner Form aus, so dass sich ein Extraktions- oder Fällungsverfahren zur Isolation dieser Verbindungen im allgemeinen erübrigt. Fällt das Produkt nicht direkt an, so kann es durch einfaches Abdampfen des Lösungsmittels erhalten werden.

Einige der Phosphoniumsalze der Formel IV haben selbst auch fungizide Eigenschaften.

Die für die Herstellung der Phosphonate der Formel III und der Phosphoniumsalze der Formel IV eingesetzten 1-Halogenmethyl-1H-1,2,4-triazole der Formel VI sind neu. Sie bilden einen Gegenstand der Europäischen Patentanmeldung Nr. 60 222.

Die 1-Halogenmethyl-1H-1,2,4-triazole der Formel VI,

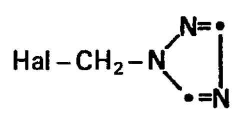 (VI),

worin Hal für Chlor, Brom oder Jod steht, werden erhalten, indem man 1-Hydroxymethyl-1H-1,2,4-triazol der Formel X

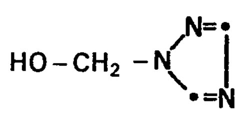 (X)

mit einem Halogenierungsmittel umsetzt und die entstandenen Hydrohalogenide der Formel XI,

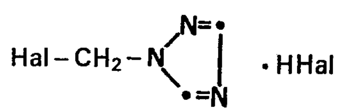 (XI),

worin Hal die unter Formel VI angegebene Bedeutung hat, mit einer Base behandelt.

Als Basen eignen sich starke anorganische Hydroxide wie Natrium- und Kaliumhydroxid.

Als Halogenierungsmittel können Verwendung finden: Phosgen, Thionylchlorid, Thionylbromid, Phosphortrichlorid, Phosphortribromid, Phosphorpentachlorid, Phosphoroxychlorid oder Jodwasserstoffsäure. Vorzugsweise werden Thionylchlorid und Thionylbromid zur Halogenierung

eingesetzt, da die entstehenden Nebenprodukte gasförmig sind, aus der Reaktionslösung entweichen und deshalb die Reaktion nicht beeinflussen. Die Jodverbindungen werden mit Vorteil aus bereits halogenierten Verbindungen erhalten, wenn man diese mit Jodwasserstoffsäure umsetzt. Eine weitere Methode zur Herstellung von Chloriden, Bromiden und Jodiden besteht in der Umsetzung von X mit den entsprechenden Trialkylsilyl-Halogeniden.

Die Halogenierungsreaktion wird in inerten Lösungsmitteln ausgeführt, wie z.B. Kohlenwasserstoffen wie Hexan, Cyclohexan, Benzol, Toluol oder Xylol oder Äthern wie Diäthyläther, Tetrahydrofuran, Dioxan oder Dimethoxyäthan. Bei Verwendung von flüssigen Halogenierungsmitteln kann häufig ganz auf ein Lösungsmittel verzichtet werden. Die Reaktion wird dann in einem Überschuss der Reagenzes, z.B. Thionylchlorid oder -bromid durchgeführt.

Dieses Herstellungsverfahren und ein neues Verfahren zur Herstellung von 1-Hydroxymethyl-1H-1,2,4-triazol (X), das aus Khim. Geterosikl. Soedi., 1980, 251 (Engl. Übersetzung: Chem. Heterocycl. Comp. 1980, 189) bekannt ist, bilden einen weiteren Aspekt der schweizerischen Patentanmeldung Nr. 1622/81.

Die 1-Halogenmethyl-1H-1,2,4-triazole sind bei Raumtemperatur und auch bei 0 °C keine stabilen Verbindungen.

Jedoch wurde überraschenderweise festgestellt, dass Lösungen von 1-Halogenmethyl-1H-1,2,4-triazolen der Formel VI in Acetonitril monatelang ohne Zersetzung haltbar sind.

Das 1-Hydroxymethyl-triazol der Formel X wird nach dem neuen Verfahren hergestellt, indem man in nicht wässrigem Medium 1H-1,2,4-Triazol in Gegenwart von katalytischen Mengen Base mit Paraformaldehyd umsetzt.

Als Lösungsmittel können bei diesem Verfahren inerte organische Verbindungen dienen wie z.B. Äther, Dimethoxyäthan, Dioxan, Tetrahydrofuran. Zur Durchführung der Reaktion wird das Reaktionsgemisch dabei am Rückfluss gekocht. Ganz besonders vorteilhaft ist es jedoch, wenn ohne Lösungsmittel in der Schmelze der Reaktionspartner gearbeitet wird. Die Temperatur wird dabei zwischen 40° und 150 °C, insbesondere zwischen 60° und 100 °C gehalten.

Als Basen finden Anwendung: Hydroxide wie Natrium- oder Kaliumhydroxid; Carbonate wie Natrium- oder Kaliumcarbonat oder Amine wie Triäthylamin, Chinolin und Pyridin.

Die als Zwischenprodukte hergestellten 2-(1H-1,2,4-Triazol-1-yl)-styrole der Formel Va sind neu. Verbindungen dieses Strukturtyps sind als Insektizide in der deutschen Offenlegungsschrift 2 833 194 beschrieben.

Die neuen 2-(1H-1,2,4-Triazol-1-yl)-styrole entsprechen der allgemeinen Formel Va,

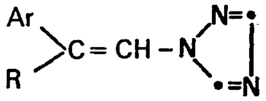 (Va),

worin

Ar einen gegebenenfalls durch $C_1$–$C_4$-Alkyl, $C_1$–$C_4$-Alkoxy, Cyan, Nitro, Trifluormethyl oder ein bis drei Halogenatome substituierten Phenylrest bedeutet und

R für $C_1$–$C_{10}$-Alkyl; $C_2$–$C_5$-Alkenyl; $C_3$–$C_8$-Cycloalkyl; gegebenenfalls durch $C_3$–$C_8$-Cycloalkyl oder Phenyl substituiertes $C_1$–$C_4$-Alkyl steht, wobei der Phenylsubstituent seinerseits gegebenenfalls durch Halogen, $C_1$–$C_4$-Alkyl oder $C_1$–$C_4$-Alkoxy substituiert sein kann.

Die Verbindungen der Formel V liegen als E–Z-Isomerengemische vor und werden, wenn nicht speziell angegeben, als solche verwendet.

Die anschliessenden Beispiele dienen der näheren Illustration der Erfindung. Temperaturen sind in Celsiusgraden, °C, Drücke in Millibar, mb, oder Bar, b, angegeben.

Beispiel 1

Herstellung eines Zwischenproduktes

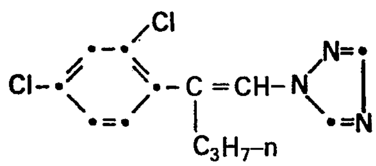 Verbindung Nr. 1)

1-(1H-1,2,4-Triazol-1-yl)-2-(2,4-dichlorphenyl)-1-pentan

a) Unter einer Stickstoffatmosphäre lässt man unter Feuchtigkeitsausschluss zur auf 5° bis 7° gekühlten Suspension von 1,6 g 55%igem Natriumhydrid in 15 ml Dimethoxyäthan 7,8 g (0,036 Mol) Diäthyl-(1H-1,2,4-triazol-1-yl)-methylphosphonat zutropfen. Nachdem nach 2 Stunden Rühren bei Zimmertemperatur die Wasserstoffentwicklung beendet ist, wird die Reaktionsmischung wieder auf 8° bis 9 °C gekühlt und tropfenweise mit einer Lösung von 6,5 g (0,03 Mol) 2,4-Dichlorphenyl-n-propylketon in 15 ml Dimethoxyäthan versetzt. Danach wird die Mischung für 18 Stunden auf 50° erwärmt, anschliessend mit Eiswasser versetzt und dreimal mit Methylenchlorid extrahiert. Die Extrakte werden mit Wasser gewaschen, getrocknet und eingedampft. Durch fraktionierte Destillation erhält man 6,5 g (76,8%) 1-(1H-1,2,4-Triazol-1-yl)-2-(2,4-di-chlorphenyl)-1-penten als E–Z-Isomerengemisch, Spd. 135–137 °C/0.42 mb.

$C_{13}H_{13}Cl_2N_3$ (282,2)

| | | | |
|---|---|---|---|
| Ber.: | C 55,34%; | H 4,65%; | N 14,89% |
| Gef.: | C 55,2 %; | H 4,7 %; | N 14,9 %. |

b) Unter einer Stickstoffatmosphäre setzt man unter Feuchtigkeitsanschluss einer Suspension von 1,0 g 55%igem Natriumhydrid in 50 ml Dimethoxyäthan in 2 Portionen insgesamt 7,6 g (0,02 Mol) kristallines 1H-1,2,4-Triazol -1-yl-methyl-triphenylphosphoniumchlorid zu. Die Reaktionsmischung nimmt eine orangerote Färbung an und die Temperatur steigt auf ca. 35° an. Nach Beendigung der Wasserstoffentwicklung lässt man noch ca. 3 Stunden 4,3 g (0,02 Mol) 2,4-Dichlorphenyl-n-propylketon in 10 ml Dimethoxyäthan zutropfen. Die Reaktionstemperatur steigt dabei auf ca. 35° an. Nach 18 Stunden Rühren wird die Reaktionsmischung mit Eiswasser versetzt und dreimal mit Diäthyläther extrahiert. Die Extrakte werden mit Wasser gewaschen, getrocknet und eingeengt. Das ausgefallene Triphenylphosphinoxid wird abgetrennt und die konzentrierte Lösung durch Filtration über eine kurze Kieselgelsäule gereinigt. Eindampfen des Filtrats ergibt 4,0 g (74,7%) 1-(1H-1,2,4-Triazol -1-yl)- 2-(2,4-dichlorphenyl) -1-penten, das nach Dünnschichtchromatogram und $^1H$-NMR-Spektrum identisch ist mit authentischem Material.

In der anschliessenden Tabelle 1 sind die nach dem Beispiel 1 hergestellte und analog herzustellende Verbindungen aufgeführt.

Tabelle 1
Verbindungen der Formel V

| Nr. | Ar | R | phys.Daten |
|---|---|---|---|
| 1 | 2-Cl-4-Cl-$C_6H_3$– | –$C_3H_7$-n | Sdp. 135–137°/0.42 mb |
| 2 | $C_6H_5$– | H– | |
| 3 | 2-Cl-4-Cl-$C_6H_3$– | H– | Sdp. 146 °C/0.6 mb |
| 4 | $C_6H_5$– | $CH_3$– | |
| 5 | 4-F–$C_6H_4$– | H–[· $HNO_3$] | Smp. 155 °C |
| 6 | 2-Cl-4-Cl–$C_6H_3$– | $CH_3$– | Öl |
| 7 | 2-Cl-4-Cl–$C_6H_3$– | i-$C_3H_7$– | |
| 8 | 2-Cl-4-Cl–$C_6H_3$– | $C_2H_5$– | |
| 9 | 2-Cl-4-Cl–$C_6H_3$– | $H_2C$=CH–$CH_2$– | |
| 10 | 2-Cl-4-Cl–$C_6H_3$– | n-$C_4H_9$– | |
| 11 | 2-Cl-4-Cl–$C_6H_3$– | $(CH_3)_2$CH–$CH_2$– | |
| 12 | 2-Cl-4-Cl–$C_6H_3$– | $CH_3$–$CH_2$–CH($CH_3$)– | |
| 13 | 2-Cl-4-Cl–$C_6H_3$– | n-$C_5H_{11}$– | |
| 14 | 2-Cl-4-Cl–$C_6H_3$– | cycl. $C_5H_9$– | |
| 15 | 2-Cl-4-Cl–$C_6H_3$– | cycl. $C_6H_{11}$– | |
| 16 | 2-Cl-4-Cl–$C_6H_3$– | 4-Cl–$C_6H_4$– | |
| 17 | 2-Br-4-Br-$C_6H_3$– | n-$C_3H_7$– | |
| 18 | 2-Br-4-Br–$C_6H_3$– | n-$C_4H_9$– | |
| 19 | 2-Br-4-Br-$C_6H_3$– | i-$C_3H_7$– | |
| 20 | 2-Br-4-Br–$C_6H_3$– | $(CH_3)_2$CH–$CH_2$– | |
| 21 | 2-Br-4-Br–$C_6H_3$– | $CH_3$–$CH_2$–CH($CH_3$)– | |
| 22 | 2-Cl-4-Cl–$C_6H_3$– | 4-Cl–$C_6H_4$–$CH_2$– | |
| 23 | 4-F–$C_6H_4$– | 4-Cl–$C_6H_4$–$CH_2$–$CH_2$– | |
| 24 | 4-F–$C_6H_4$– | 4-$CH_3$–$C_6H_4$–$CH_2$–$CH_2$– | |
| 25 | 2-Cl-4-Cl–$C_6H_3$– | cycl. $C_6H_{11}$–$CH_2$–$CH_2$– | |
| 26 | 2-Cl-4-Cl–$C_6H_3$– | cycl. $C_5H_9$–$CH_2$–$CH_2$– | |
| 27 | 2-Cl-4-Cl–$C_6H_3$– | 4-$CH_3$-3-Cl–$C_6H_3$–$CH_2$– | |
| 28 | $C_6H_5$– | cycl. $C_3H_5$– | Öl |
| 29 | 4-Cl–$C_6H_4$– | cycl. $C_6H_{11}$– | Öl |
| 30 | 4-$OCH_3$–$C_6H_4$– | $C_2H_5$– | Öl |
| 31 | 4-Cl–$C_6H_4$– | –$C_3H_7$-n | Öl |
| 32 | 4-$OCH_3$–$C_6H_4$– | cycl. $C_3H_5$– | Smp. 82–83 °C |
| 33 | 4-Cl–$C_6H_4$– | cycl. $C_3H_5$– | Smp. 60–62 °C |
| 34 | 3-Cl-4-Cl–$C_6H_3$– | $CH_3$ | halbfest |
| 35 | 2-Cl-4-Cl–$C_6H_3$– | –$C_3H_7$-n- | Smp. 64–66 °C (E-Isomeres) |

Beispiel 2

Herstellung eines Endproduktes

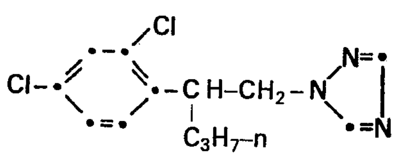

1-(1H-1,2,4-Triazol-1-yl)-2-(2,4-dichlorphenyl)-pentan

1,4 g (0,005 Mol) 1-(1H-1,2,4-Triazol -1-yl)-2- (2,4-dichlorphenyl) -1-penten werden in 150 ml Tetrahydrofuran an einem Gemisch von 0,3 g Rhodiumoxid/Platinoxid-Katalysator (1:1 und 0,3 g Rhodium/Kohle-Katalysator in 5,5 Stunden bei 20° und 1 b hydriert. Nachdem das Katalysatorengemisch abgetrennt, die Lösung mit Bleicherde behandelt und das Lösungsmittel abgedampft worden ist, wird das ölige Produkt mit Äther an Kieselgel chromatographiert. Man erhält 1,0 g (71,4%) (1-(1H-1,2,4-Triazol -1-yl)-2-(2,4-dichlorphenyl-pentan als farbloses Öl, das aus Äther/Petroläther kristallisiert, einen Smp. von 58,5°–59,5° aufweist.

$C_{13}H_{14}Cl_2N_3$ (284,2)
Ber.: C 54,95%; H 5,32%; N 14,79%;
Gef.: C 54,7 %; H 5,6 %; N 14,4 %.

Beispiel 3
Herstellung eines Ausgangsproduktes

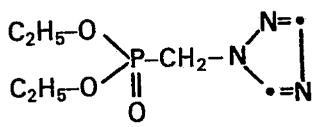

Diäthyl-(1H-1,2,4-triazol-1-yl)-methylphosphonat

a) Zur Dispersion von 17,6 g (0,1 Mol) Kaliumdiäthylphosphit in 100 ml Toluol lässt man bei 90° 11,7 g (0,1 Mol) frisch hergestelltes 1-Chlormethyl-1H-1,2,4-triazol zutropfen. Nach Beendigung der exothermen Reaktion wird das ausgefallene Kaliumchlorid abgetrennt und das Filtrat eingedampft. Die Chromatographie des Rückstands mit Äthylacetat an Kieselgel ergibt 2,1 g (9,6%) gelbes Öl. Durch Kugelrohrdestillation dieses Öls erhält man Diäthyl-(1H-1,2,4-triazol -1- yl)-methylphosphonat als farbloses Öl, Sdp. 130°/0,11 mb.

$^1$H–NMR ($CDCl_3$, TMS): $\delta$ = 8,4 (s; 1H, Triazol 5-CH); 8,07 (s; 1H, Triazol 3-CH); 4,75 (d, $J_{PCH}$ = 13 Hz; 2H, P–$CH_2$); 4,25 (2q, J = 7 Hz; 4H, $OCH_2$) und 1,5 (t, J = Hz; 6H, $CH_3$) ppm.

$^{31}$P–NMR ($CD_3OD$, $H_3PO_4$): $\delta$ = + 17,12 ppm.

b) Eine Mischung aus 18,2 g (0,155 Mol) 1-Chlormethyl-1H-1,2,4-triazol, 27,2 g (0,17 Mol) Natrium-diäthylphosphit und 200 ml Acetonitril wird für 2 Stunden auf 60° erwärmt und anschliessend für 16 Stunden am Rückfluss gekocht. Nach Abtrennen des Niederschlages wird die Lösung eingedampft, und fraktioniert destilliert. Man erhält 19,1 g (56,5%) Diäthyl-(1H-1,2,4-triazol-1-yl)-methylphosphonat als farbloses Öl, Sdp. 120–129°/0,053 mb.

$C_7H_{14}N_3O_3P$ (219,2)
Ber.: C 38,36%; H 6,44%; N 19,17%;
Gef.: C 38,37%; H 6,60%; N 19,76%.

c) 42 g (0,357 Mol) 1-Chlormethyl-1H-1,2,4-triazol werden in 124 ml Triäthylphosphit für 2 Stunden am Rückfluss gekocht. Nach Beendigung der Äthylchloridentwicklung wird das überschüssige Triäthylphosphit abdestilliert und der Rückstand durch eine Hochvakuumkurzwegdestillation gereinigt. Man erhält 23,1 g (29,5%) Diäthyl-(1H-1,2,4-triazol-1-yl)-methyl-phosphonat, Sdp. 130°/0,11 mb.

d) Zur siedenden Mischung von 0,2 g Palladiumchlorid, 124 ml (0,715 Mol) Triäthylphosphit und 200 ml Acetonitril lässt man eine Lösung von 42 g (0,357 Mol) 1-Chlormethyl-1H-1,2,4-triazol in 30 ml Acetonitril zutropfen und kocht die Mischung für weitere 20 Stunden am Rückfluss. Nachdem überschüssiges Acetonitril und Triäthylphosphit abgedampft worden sind, wird der Rückstand durch eine Hochvakuumkurzwegdestillation gereinigt. Man erhält 5,4 g (5,9%) Diäthyl- (1H-1,2,4-triazol-1-yl)- methylphosphonat, Sdp. 130°/0,11 mb.

Beispiel 4
Herstellung eines weiteren Ausgangsproduktes

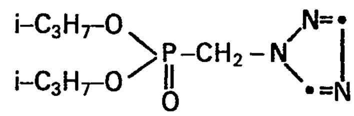

Di-i-propyl-(1H-1,2,4-triazol-1-yl)-methylphosphonat

Zur Dispersion von 18,8 g (0,1 Mol) Natriumdi-i-propylphosphit in 100 ml Toluol lässt man bei 90° 11,7 g (0,1 Mol) frisch hergestelltes 1-Chlormethyl-1H-1,2,4-triazol zutropfen. Nach Beendigung der exothermen Reaktion wird das ausgefallene Natriumchlorid abgetrennt und das Filtrat eingedampft. Die Chromatographie des Rückstands mit Äthylacetat an Kieselgel ergibt 2,9 g (11,7%) gelbes Öl. Durch Kugelrohrdestillation dieses Öls erhält man Di-i-propyl-(1H-1,2,4-triazol-1-yl)-methylphosphonat als farbloses Öl.

$^1$H–NMR ($CDCl_3$, TMS): $\delta$ = 8,4 (s; 1H, 5-CH); 7,93 (s; 1H, 3-CH); 4,6 (m, 2H, O–CH); 4,5 (d, $J_{PCH}$ = 13 Hz; 2H, P–$CH_2$) und 1,2 (d, J= 7 Hz; 12H, $CH_3$) ppm.

Beispiel 5
Herstellung eines weiteren Ausgangsproduktes

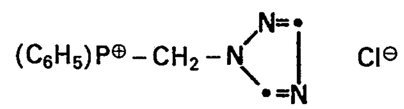

1H-1,2,4-Triazol-1-yl-methyl-triphenyl-phosphoniumchlorid

Eine Lösung von 40,6 g (0,345 Mol) 1-Chlormethyl-1H-1,2,4-triazol und 90,6 (0,345 Mol) Triphenylphosphin in 250 ml Acetonitril wird für 16 Stunden zum Rückfluss erhitzt. Beim Abkühlen der Lösung scheiden sich 91,7 g (70,0%) 1H-1,2,4-Triazol-1-yl-methyl-triphenyl-phosphoniumchlorid als farblose Kristalle ab, Smp. 287°.

$C_{21}H_{19}ClN_3P$ (379,8)
Ber.: C 66,41%; H 5,04%; N 11,06%; Cl 9,34%;
Gef.: C 64,56%; H 5,04%; N 11,38%; Cl 9,68%.

$^1$H–NMR ($CD_3COOD$): $\delta$ = 9,7 (s; 1H, 5-CH); 8,4 (s; 1H, 3-CH); 7,5 (m; 15H, $C_6H_5$) und 6,3 (d, $J_{PCH}$ = 7Hz; 2H, P–$CH_2$) ppm.

Beispiel 6
Herstellung eines weiteren Ausgangsproduktes

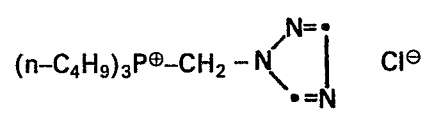

1H-1,2,4-Triazol-1-yl-methyl-tri-n-butylphosphoniumchlorid

Eine Lösung von 3,53 g (0,03 Mol) 1-Chlormethyl-1H-1,2,4-triazol und 6,1 g (0,03 Mol) Tributylphosphin in 30 ml Acetonitril wird für 16 Stunden zum Rückfluss erhitzt. Nach dem Abdampfen des Lösungsmittels erhält man 9,7 g (100%) 1H-1,2,4-Triazol-1-yl-methyl-tri-n-butyl- phosphoniumchlorid als wachsartige Masse.

$^1H$-NMR ($CD_3OC$): $\delta$ = 8,7 (s; 1H, 5-CH); 7,8 (s; 1H, 3-CH); 5,5 (d, $J_{PCH}$ = 7Hz; 2H, P–$CH_2$) und 0,5–2,7 (m; 27H, $C_4H_9$) ppm.

Beispiel 7

Herstellung eines weiteren Ausgangsproduktes

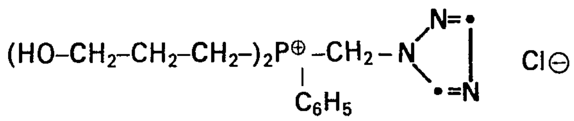

1H-1,2,4-Triazol-1-yl-methyl-di-(3-hydroxypropyl)-phenylphosphoniumchlorid

Eine Lösung von 3,53 g (0,03 Mol) 1-Chlormethyl-1H-1,2,4-triazol und 6,88 g (0,03 Mol) Di-(3-hydroxypropyl)-phenyl-phosphin in 50 ml Acetonitril wird für 16 Stunden zum Rückfluss erhitzt. Nach dem Abdampfen des Lösungsmittels erhält man 10 g (98,5%) 1H-1,2,4-Triazol -1-yl-methyl-di- (3-hydroxypropyl) -phenylphosphoniumchlorid als hochviskoses Öl.

$^1H$-NMR ($CD_3OD$): $\delta$ = 8,2 (s; 1H, 5-CH); 7,7 (s; 1H, 3-CH); 7,4 (m; 5H, $C_6H_5$); 5,67 (d, $J_{PCH}$ = 7Hz; 2H, P–$CH_2$–N); 4,3 (s; 2H, OH); 3,3 (m; 4H, O–$CH_2$); 2,6 (m; 4H, P–$CH_2$) und 1,6 (m; 4H, $CH_2$) ppm.

Beispiel 8

Herstellung eines Vorproduktes

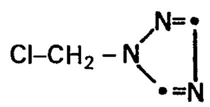

1-Chlormethyl-1H-1,2,4-triazol

a) Beim Erhitzen einer Mischung von 140 g (2,0 Mol) 1H-1,2,4-Triazol, 60 (2,0 Mol) Paraformaldehyd und 1,5 ml Triäthylamin für 2 Stunden entsteht eine klare Schmelze, die beim Abkühlen erstarrt. Man erhält durch Umkristallisieren aus Aceton 195,3 g (98,5%) 1-Hydroxymethyl-1H-1,2,4-triazol, Smp. 65–67°.

$C_3H_5N_3O$ (99,1)

| | | | |
|---|---|---|---|
| Ber.: | C 36,3%; | H 5,0%; | N 42,4%; |
| Gef.: | C 36,0%; | H 5,0%; | N 42,4%, |

$^1H$-NMR ($CDCl_3$): $\delta$ = 8,47 (s; 1H, 5-CH); 8,0 (s; 1H, 3-CH); 7,35 (s; 1H, OH) und 5,67 (s; 2H, $CH_2O$) ppm.

b) Zu 600 ml Thionylchlorid lässt man innerhalb von 1,5 Stunden eine Schmelze von 195,3 g (1,975 Mol) 1-Hydroxymethyl-1H-1,2,4-triazol zutropfen. Es setzt eine starke Gasentwicklung ein und die Reaktionsmischung beginnt zu sieden. Nachdem die Schmelze vollständig zugesetzt worden ist, wird unter Kochen am Rückfluss noch für 2 Stunden gerührt. Nach dem Abkühlen wird das ausgefallene, gelbe 1-Chlormethyl-1H-1,2,4-triazol-hydrochlorid abfiltriert und zweimal mit je 300 ml Diäthyläther gewaschen. Ausbeute 272 g (90,2%), Smp. 120–127°.

$C_3H_4ClN_3 \cdot HCl$ (154)

| | | | |
|---|---|---|---|
| Ber.: | C 23,40%; | H 3,27%; | N 27,29%; |
| Gef.: | C 23,40%; | H 3,5 %; | N 27,30%, |

$^1H$-NMR ($CD_3OD$): $\delta$ = 9,77 (s; 1H, 5-CH); 8,55 (s; 1H, 3-CH); 5,9 (s; 2H, $CH_2$–Cl) und 5,06 (s; 1H, HCl) ppm.

C) Zu 240,6 g (1,56 Mol) 1-Chlormethyl-1H-1,2,4-triazol-hydrochlorid in einem Gemisch von 600 ml Wasser und 400 ml Chloroform lässt man unter kräftigem Rühren 80 g Natriumhydroxid in 250 ml Wasser zutropfen. Nach Sättigen mit Natriumchlorid wird dreimal mit Chloroform extrahiert. Durch Trocknen, Eindampfen der Chloroformextrakte und fraktionierte Destillation des Rückstandes erhält man 156,2 g (85,1% 1-Chlormethyl-1H-1,2,4-triazol, Sdp. 52–54°/0,27 mb.

$^1H$-NMR ($CDCl_3$): $\delta$ = 8,8 (s; 1H, 5-CH); 8,3 (s; 1H, 3-CH) und 6,3 (s; 2H, $CH_2Cl$) ppm.

**Patentansprüche**

1. Verfahren zur Herstellung von 1-(2-Phenyläthyl)-1H-1,2,4-triazolen der allgemeinen Formel I,

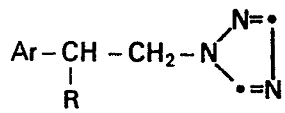

(I),

worin

Ar einen gegebenenfalls durch $C_1$–$C_4$-Alkyl, $C_1$–$C_4$-Alkoxy, Cyan, Nitro, Trifluormethyl oder ein bis drei Halogenatome substituierten Phenylrest bedeutet und

R für Wasserstoff; $C_1$–$C_{10}$-Alkyl; $C_2$–$C_5$-Alkenyl; $C_3$–$C_8$-Cycloalkyl; gegebenenfalls durch $C_3$–$C_8$-Cycloalkyl oder Phenyl substituiertes $C_1$–$C_4$-Alkyl steht, wobei der Phenylsubstituent seinerseits gegebenenfalls durch Halogen, $C_1$–$C_4$-Alkyl oder $C_1$–$C_4$-Alkoxy substituiert sein kann, dadurch gekennzeichnet, dass man ein Phenylketon der Formel II,

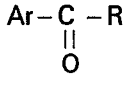

(II),

worin Ar und R die unter Formel I angegebene Bedeutung haben, zunächst durch Umsetzung mit einem 1H-1,2,4-Triazol-1-yl-methylphosphonat der Formel III,

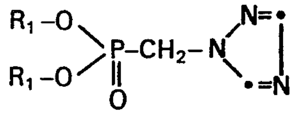

(III),

worin die Reste $R_1$ unabhängig voneinander Phenyl oder $C_1$–$C_4$-Alkyl bedeuten, oder mit einem Phosphoniumsalz der Formel IV,

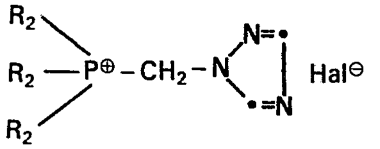

(IV),

worin die Reste $R_2$ unabhängig voneinander Phenyl oder gegebenenfalls durch Hydroxyl substituiertes $C_1$–$C_4$-Alkyl bedeuten und Hal für Chlor, Brom oder Jod steht, in einem inerten organischen Lösungsmittel in Gegenwart einer starken Base zu einem 2-(1H-1,2,4-Triazol-1-yl)-styrol der Formel V,

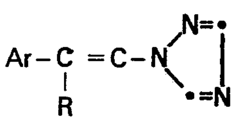

(V),

worin Ar und R die unter Formel I angegebenen Bedeutungen haben, umsetzt und dieses anschliessend durch katalytische Hydrierung in die Verbindungen der Formel I überführt.

2. Verfahren gemäss Anspruch 1, dadurch gekennzeichnet, dass man zur Herstellung der 2-(1H-1,2,4-Triazol-1-yl)-styrole der Formel V die Phosphonate der Formel III verwendet.

3. Verfahren gemäss Anspruch 1, dadurch gekennzeichnet, dass man zur Herstellung der 2-(1H-1,2,4-Triazol-1-yl)-styrole der Formel V die Phosphoniumsalze der Formel IV verwendet.

4. Verfahren gemäss Anspruch 1, dadurch gekennzeichnet, dass die katalytische Hydrierung an einem Edelmetallkatalysator erfolgt.

5. Verfahren gemäss Anspruch 4, dadurch gekennzeichnet, dass man als Katalysatoren Edelmetalloxide verwendet.

6. Verfahren gemäss einem der Ansprüche 4 oder 5, dadurch gekennzeichnet, dass man Gemische von Katalysatoren verwendet.

7. Verfahren gemäss Anspruch 6, dadurch gekennzeichnet, dass man ein Rhodiumoxid/Platinoxid-Gemisch als Katalysator verwendet.

8. Verfahren gemäss einem der Ansprüche 1 bis 3, dadurch gekennzeichnet, dass die katalytische Hydrierung in neutralem oder saurem Medium erfolgt.

9. Verfahren gemäss Anspruch 8, dadurch gekennzeichnet, dass das saure Medium durch Zusatz einer Mineralsäure erzeugt wird.

10. Verfahren gemäss Anspruch 8, dadurch gekennzeichnet, dass in Essigsäure oder Trifluoressigsäure hydriert wird.

11. Verfahren gemäss einem der Ansprüche 1 bis 3, dadurch gekennzeichnet, dass die katalytische Hydrierung bei Temperaturen zwischen 0° und 100 °C durchgeführt wird.

12. Verfahren gemäss Anspruch 11, dadurch gekennzeichnet, dass die Temperatur 10° bis 30 °C beträgt.

13. Verfahren gemäss einem der Ansprüche 1 bis 3, dadurch gekennzeichnet, dass die katalytische Hydrierung bei Drücken von 1 bis 100 Bar durchgeführt wird.

14. Verfahren gemäss Anspruch 13, dadurch gekennzeichnet, dass der Druck zwischen 1 und 8 Bar beträgt.

15. Verfahren gemäss Anspruch 2, dadurch gekennzeichnet, dass bei der Umsetzung des Phenylketons der Formel II mit dem 1H-1,2,4-Triazolyl-methylphosphonat der Formel III zunächst das Methylphosphonat mit einer starken Base und danach mit dem Phenylketon umgesetzt wird.

16. Verfahren gemäss Anspruch 2, dadurch gekennzeichnet, dass bei der Umsetzung des Phenylketons der Formel II mit dem 1H-1,2,4-Triazolyl-methylphosphonat der Formel III eine Lösung des Gemisches von Phosphonat und Phenylketon zu einer Lösung oder einer Suspension einer starken Base zugesetzt wird.

17. Verfahren gemäss Anspruch 15 oder 16, dadurch gekennzeichnet, dass die Base aus der Gruppe: metallorganische Verbindungen, Alkoholate, Metallhydride oder Alkaliamide ausgewählt wird.

18. Verfahren gemäss Anspruch 17, dadurch gekennzeichnet, dass die Basen Metallhydride oder metallorganische Verbindungen sind.

19. Verfahren gemäss Anspruch 15 oder 16 und einem der Ansprüche 17 oder 18, dadurch gekennzeichnet, dass man einen Überschuss an Base (bis zur doppelten molaren Menge) einsetzt.

20. Verfahren gemäss Anspruch 15 oder 16, dadurch gekennzeichnet, dass die Temperaturen bei der Umsetzung des Phosphonats der Formel III mit der Base –40° bis +40 °C und bei der weiteren Umsetzung mit dem Keton der Formel II –20° bis +80 °C betragen.

21. Verfahren gemäss Anspruch 20, dadurch gekennzeichnet, dass die Temperaturen im ersten Reaktionsschritt –20° bis +40 °C und im zweiten 0° bis +60 °C betragen.

22. Verfahren gemäss Anspruch 15 oder 16, dadurch gekennzeichnet, dass man die Reaktion in einem inerten, polaren, aprotischen Lösungsmittel ausführt.

23. Verfahren gemäss Anspruch 22, dadurch gekennzeichnet, dass die Lösungsmittel Äther, Säureamide oder Sulfoxide sind.

24. Verfahren gemäss Anspruch 3, dadurch gekennzeichnet, dass man bei der Umsetzung des Phenylketons der Formel II mit dem 1H-1,2,4-Triazolyl-methylphosphoniumsalz der Formel IV zunächst das Methylphosphoniumsalz mit einer starken Base und danach mit dem Phenylketon umsetzt.

25. Verfahren gemäss Anspruch 24, dadurch gekennzeichnet, dass die Base aus der Gruppe: metallorganische Verbindungen, Alkoholate, Metallhydride oder Alkaliamide ausgewählt wird.

26. Verfahren gemäss Anspruch 25, dadurch gekennzeichnet, dass die Basen metallorganische Verbindungen oder Metallhydride sind.

27. Verfahren gemäss Anspruch 24 und einem der Ansprüche 25 oder 26, dadurch gekennzeichnet, dass man einen Überschuss an Base (bis zur doppelten molaren Menge) einsetzt.

28. Verfahren gemäss Anspruch 24, dadurch gekennzeichnet, dass die Temperaturen bei der Umsetzung des Phosphoniumsalzes der Formel

IV mit der Base −40° bis +80 °C und bei der weiteren Umsetzung mit dem Keton der Formel II −20° bis +110 °C betragen.

29. Verfahren gemäss Anspruch 28, dadurch gekennzeichnet, dass die Temperaturen im ersten Reaktionsschritt −20° bis +40 °C und im zweiten 0° bis 60 °C betragen.

30. Verfahren gemäss Anspruch 24, dadurch gekennzeichnet, dass man die Reaktion in einem inerten, polaren, aprotischen Lösungsmittel ausführt.

31. Verfahren gemäss Anspruch 30, dadurch gekennzeichnet, dass die Lösungsmittel Äther, Säureamide oder Sulfoxide sind.

32. Verfahren gemäss Anspruch 2, dadurch gekennzeichnet, dass man bei der Umsetzung des Phenylketons der Formel II mit dem Methylphosphonat der Formel III zunächst das Phosphonat mit einem Überschuss (bis zur doppelten molaren Menge) einer Base, ausgewählt aus der Gruppe metallorganische Verbindungen, Alkoholate, Metallhydride oder Alkaliamide, in einem inerten, polaren, aprotischen Lösungsmittel bei einer Temperatur von −40° bis +40 °C und dann mit dem Keton der Formel II bei Temperaturen zwischen −20° und +80 °C zum Triazolylstyrol der Formel V umsetzt und dieses ananschliessend mit einem Edelmetallkatalysator in neutralem oder saurem Medium bei Temperaturen von 0° bis 100 °C und Drücken von 1 bis 100 Bar hydriert.

33. Verfahren gemäss Anspruch 32, dadurch gekennzeichnet, dass man für die Umsetzung des Phosphonats als Base ein Metallhydrid oder eine metallorganische Verbindung, als Lösungsmittel Äther, Säureamide oder Sulfoxide verwendet und die Temperatur zwischen −20° und +40 °C wählt; die Umsetzung mit dem Keton bei Temperaturen zwischen 0° und 60 °C ausführt und das Triazolylstyrol mit einem Rhodiumoxid/Platinoxid-Gemisch bei 10° bis 30 °C und 1 bis 8 Bar hydriert.

34. Verfahren gemäss Anspruch 3, dadurch gekennzeichnet, dass man bei der Umsetzung des Phenylketons der Formel II mit dem Methylphosphoniumsalz der Formel IV zunächst das Phosphoniumsalz mit einem Überschuss (bis zur doppelten molaren Menge) einer Base, ausgewählt aus der Gruppe metallorganische Verbindungen, Alkoholate, Metallhydride oder Alkaliamide, in einem inerten, polaren, aprotischen Lösungsmittel bei einer Temperatur von −40° bis +80 °C und dann mit dem Keton der Formel II bei Temperaturen zwischen −20° und +110 °C zum Triazolylstyrol der Formel V umsetzt und dieses anschliessend mit einem Edelmetallkatalysator in neutralem oder saurem Medium bei Temperaturen von 0° bis 100 °C und Drücken von 1 bis 100 Bar hydriert.

35. Verfahren gemäss Anspruch 34, dadurch gekennzeichnet, dass man für die Umsetzung des Phosphonats als Base ein Metallhydrid oder eine metallorganische Verbindung, als Lösungsmittel Äther, Säureamide oder Sulfoxide verwendet und die Temperatur zwischen −20° und +40 °C wählt; die Umsetzung mit dem Keton bei Temperaturen zwischen 0° und 60 °C ausführt und das Triazolylstyrol mit einem Rhodiumoxid/Platinoxid-Gemisch bei 10° bis 30 °C und 1 bis 8 Bar hydriert.

36. 1H-1,2,4-Triazol-1-yl-methylphosphonate der allgemeinen Formel III,

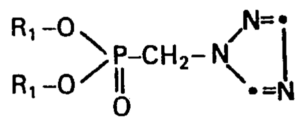

(III),

worin die Reste $R_1$ unabhängig voneinander Phenyl oder $C_1$–$C_4$-Alkyl bedeuten.

37. 1H-1,2,4-Triazol-1-yl-methylphosphoniumsalze der allgemeinen Formel IV,

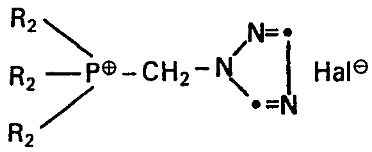

(IV),

worin die Reste $R_2$ unabhängig voneinander Phenyl oder gegebenenfalls durch Hydroxyl substituiertes $C_1$–$C_4$-Alkyl und Hal Chlor, Brom oder Jod bedeuten.

38. Verfahren zur Herstellung der Verbindungen der Formel III, gemäss Anspruch 36, dadurch gekennzeichnet, dass man ein 1-Halogenmethyl-1H-1,2,4-triazol der Formel VI,

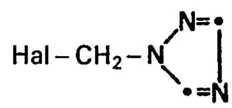

(VI),

worin Hal für Chlor, Brom oder Jod steht, entweder mit einem sekundären Phosphit der Formel VII,

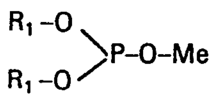

(VII),

worin $R_1$ die im Anspruch 36 gegebene Bedeutung hat und Me für ein Alkalimetallatom steht, oder mit einem tertiären Phosphit der Formel VIII umsetzt,

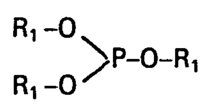

(VIII),

worin $R_1$ die in Anspruch 36 gegebene Bedeutung hat.

39. Verfahren zur Herstellung der Verbindungen der Formel IV, gemäss Anspruch 37, dadurch gekennzeichnet, dass man ein 1-Halogenmethyl-1H-1,2,4-triazol der Formel VI mit einem Phosphin der Formel IX umsetzt,

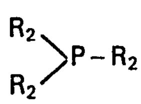

(IX),

worin $R_2$ die in Anspruch 37 angegebene Bedeutung hat.

40. Verfahren gemäss Anspruch 38, dadurch gekennzeichnet, dass man in einem inerten organischen Lösungsmittel das sekundäre Phosphit der Formel VII bei 50° bis 150 °C oder das tertiäre Phosphit der Formel VIII bei 100° bis 180 °C mit dem Halogenmethyltriazol der Formel VI umsetzt.

41. Verfahren gemäss Anspruch 40, dadurch gekennzeichnet, dass die Reaktionstemperatur für das sekundäre Phosphit zwischen 80° und 120 °C und für das tertiäre Phosphit zwischen 120° und 160 °C liegt.

42. Verfahren gemäss Anspruch 39, dadurch gekennzeichnet, dass die Reaktion in einem inerten organischen Lösungsmittel bei 30° bis 120 °C durchgeführt wird.

43. Verfahren gemäss Anspruch 42, dadurch gekennzeichnet, dass die Reaktionstemperatur zwischen 50° und 100 °C liegt.

**Revendications**

1. Procédé de préparation de 1-(2-phényléthyl)-1 H-1,2,4-triazoles de formule générale I,

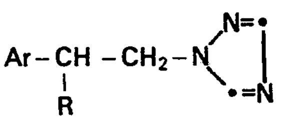

(I),

où

Ar représente un reste phényle substitué éventuellement par un alkyle $C_1$–$C_4$, un alcoxy $C_1$–$C_4$, un cyan, un nitro, un trifluorométhyle ou par un à trois atomes d'halogène et

R représente un hydrogène; un alkyle $C_1$–$C_{10}$, un alkényl $C_3$–$C_8$; un alkyle $C_1$–$C_4$ substitué éventuellement par un cycloalkyle $C_3$–$C_8$ ou un phényle, le substituant phényle pouvant de son côté être éventuellement substitué par un halogène, un alkyle $C_1$–$C_4$ ou un alcoxy $C_1$–$C_4$, caractérisé en ce que on transforme une phénylcétone de la formule II,

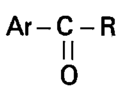

(II),

où Ar et R ont la signification indiquée sous la formule I, en premier lieu par réaction avec un 1 H-1,2,4-triazol-1-yl-méthyl-phosphonate de la formule III,

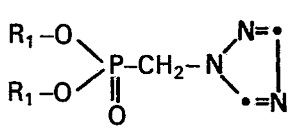

(III),

où les restes $R_1$ représentent indépendamment l'un de l'autre un phényle ou un alkyle $C_1$–$C_4$, ou avec un sel de phosphonium de la formule IV,

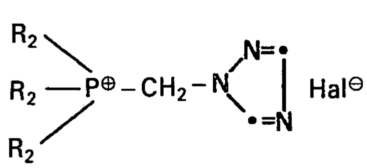

(IV),

où les restes $R_2$ représentent indépendamment l'un de l'autre un phényle ou un alkyle $C_1$–$C_4$ éventuellement substitué par un hydroxyle et Hal représente un chlore, un brome ou un iode, dans un solvant organique inerte en présence d'une base forte, en un 2-(1H-1,2,4-triazol-1-yl)-styrène de la formule V,

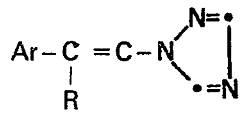

(V),

où Ar e R ont les significations indiquées sous la formule I et on transforme ensuite celui-ci en composés de la formule I.

2. Procédé selon la revendication 1, caractérisé en ce que pour la préparation des 2-(1H-1,2,4-triazol-1-yl)-styrènes de la formule V on utilise les phosphonates de la formule III.

3. Procédé selon la revendication 1, caractérisé en ce que pour la préparation des 2-(1H-1,2,4-triazol-1-yl)-styrènes de la formule V, on utilise les sels de phosphonium de la formule IV.

4. Composé selon la revendication 1, caractérisé en ce que l'hydrogénation catalytique s'effectue sur un catalyseur de métal précieux.

5. Procédé selon la revendication 4, caractérisé en ce qu'on utilise comme catalyseurs des oxydes de métaux précieux.

6. Procédé selon l'une des revendications 4 ou 5, caractérisé en ce qu'on utilise un mélange de catalyseurs.

7. Procédé selon la revendication 6, caractérisé en ce qu'on utilise comme catalyseur un mélange oxyde de rhodium/oxyde de platine.

8. Procédé selon l'une des revendications 1 à 3, caractérisé en ce que l'hydrogénation catalytique s'effectue en milieu neutre ou acide.

9. Procédé selon la revendication 8, caractérisé en ce que le milieu acide est obtenu par addition d'un acide minéral.

10. Procédé selon la revendication 8, caractérisé en ce que l'hydrogénation se fait dans l'acide acétique ou l'acide trifluoracétique.

11. Procédé selon l'une des revendications 1 à 3, caractérisé en ce que l'hydrogénation catalytique s'effectue à des températures de 0° à 100 °C.

12. Procédé selon la revendication 11, caractérisé en ce que la température se situe entre 10° et 30 °C.

13. Procédé selon l'une des revendications 1 à 3, caractérisé en ce que l'hydrogénation catalytique s'effectue sous des pressions de 1 à 100 bar.

14. Procédé selon la revendication 13, caractérisé en ce que la pression se situe entre 1 et 8 bar.

15. Procédé selon la revendication 2, caractérisé en ce que, lors de la réaction de la phénylcétone de la formule II avec le 1H-1,2,4-triazolylméthylphosphonate de la formule III, on fait réagir d'abord le phosphonate de méthyle avec une base forte, puis avec la phénylcétone.

16. Procédé selon la revendication 2, caractérisé en ce que lors de la réaction de la phénylcétone de la formule II avec 1H-1,2,4-triazolyl-méthyl-

phosphonate de la formule III, une solution du mélange de phosphonate et de phénylcétone est ajoutée à une solution ou une suspension d'une base forte.

17. Procédé selon la revendication 15 ou 16, caractérisé en ce que la base est choisie dans le groupe: composés organométalliques, alcoolates, hydrures de métaux ou alcali-amides.

18. Procédé selon la revendication 17, caractérisé en ce que les bases sont des hydrures de métaux ou des composés organométalliques.

19. Composé selon la revendication 15 ou 16 et l'une des revendications 17 ou 18, caractérisé en ce qu'on utilise un excès de base (jusqu'à une quantité molaire double).

20. Procédé selon la revendication 15 ou 16, caractérisé en ce que les températures, lors de la réaction du phosphonate de la formule III avec la base, se situent entre −40° et +40 °C et, lors de la réaction ultérieure avec la cétone de la formule II, entre −20° et +80 °C.

21. Procédé selon la revendication 20, caractérisé en ce que les températures, dans la première étape réactionnelle, se situent entre −20° et +40 °C, dans la deuxième, entre 0° et +60 °C.

22. Procédé selon la revendication 15 ou 16, caractérisé en ce que la réaction est réalisée dans un solvant aprotique, polaire, inerte.

23. Procédé selon la revendication 22, caractérisé en ce que les solvants sont des éthers, des amides d'acides ou des sulfoxydes.

24. Procédé selon la revendication 3, caractérisé en ce que lors de la réaction de la phénylcétone de la formule II avec le sel de 1H-1,2,4-triazolyl-méthylphosphonium de la formule IV, on fait réagir en premier lieu le sel de méthylphosphonium avec une base forte, puis avec la phénylcétone.

25. Procédé selon la revendication 24, caractérisé en ce que la base est choisie dans le groupe: composés organométalliques, alcoolates, hydrures de métaux ou alcali-amides.

26. Procédé selon la revendication 25, caractérisé en ce que les bases sont des composés organométalliques ou des hydrures de métaux.

27. Procédé selon la revendication 24 et l'une des revendications 25 ou 26, caractérisé en ce qu'on utilise un excès de base (jusqu'à la quantité molaire double).

28. Procédé selon la revendication 24, caractérisé en ce que les températures, lors de la réaction du sel de phosphonium de la formule IV avec la base, se situent entre −40° et +80 °C et, lors de la réaction ultérieure avec la cétone de la formule II, entre −20° et +110 °C.

29. Procédé selon la revendication 28, caractérisé en ce que les températures, dans la première étape réactionnelle, se situent entre −20° et +40 °C et, dans la deuxième, entre 0° et 60 °C.

30. Procédé selon la revendication 24, caractérisé en ce que la réaction est réalisée dans un solvant aprotique, polaire, inerte.

31. Procédé selon la revendication 30, caractérisé en ce que les solvants sont des éthers, des amides d'acides ou des sulfoxydes.

32. Procédé selon la revendication 2, caractérisé en ce que, lors de la réaction de la phénylcétone de la formule II avec le phosphonate de méthyle de la formule III, on fait réagir d'abord le phosphonate avec un excès (jusqu'à une quantité molaire double) de base choisie dans le groupe: composés organométalliques, alcoolates, hydrures de métaux ou alcali-amides, dans un solvant aprotique, polaire, inerte, à une température entre −40° et +40 °C, puis avec la cétone de la formule II, à des températures entre −20° et +80 °C, pour obtenir le triazolylstyrène de la formule V et ce dernier est ensuite hydrogéné avec un catalyseur de métal précieux en milieu neutre ou acide à des températures de 0° à 100 °C et sous des pressions de 1 à 100 bar.

33. Procédé selon la revendication 32, caractérisé en ce que pour la réaction du phosphonate, on utilise comme base un hydrure de métal ou un composé organométallique, comme solvants des éthers, des amides d'acides ou des sulfoxydes et on choisit la température entre −20° et +40 °C; qu'on effectue la réaction avec la cétone à des températures entre 0° et 60 °C et que l'hydrogénation du triazolylstyrène se fait avec un mélange oxyde de rhodium/oxyde de platine entre 10° et 30 °C et entre 1 à 8 bar.

34. Procédé selon la revendication 3, caractérisé en ce que lors de la réaction de la phénylcétone de la formule II avec le sel de méthylphosphonium de la formule IV, on fait réagir en premier lieu le sel de phosphonium avec un excès (jusqu'à la quantité molaire double) de base choisie dans le groupe composés organométalliques, alcoolates, hydrures de métaux ou alcaliamides, dans un solvant aprotique, polaire, inerte, à une température entre −40° et +80 °C, puis avec la cétone de la formule II, à des températures entre −20° et +110 °C, pour obtenir le triazolylstyrène de la formule V, et ce dernier est ensuite hydrogéné avec un catalyseur de métal précieux en milieu neutre ou acide à des températures entre 0° et 100 °C et des pressions entre 1 et 100 bar.

35. Procédé selon la revendication 34, caractérisé en ce que pour la réaction du phosphonate, on utilise comme base un hydrure de métal ou un composé organométallique, comme solvants des éthers, des amides d'acides ou des sulfoxydes et qu'on choisit la température entre −20° et +40 °C; qu'on effectue la réaction avec la cétone à des températures entre 0° et 60 °C et l'hydrogénation du triazolylstyrène avec un mélange oxyde de rhodium/oxyde de platine entre 10° et 30 °C et 1 à 8 bar.

36. 1H-1,2,4-triazol-1-yl-méthylphosphonate de formule générale III,

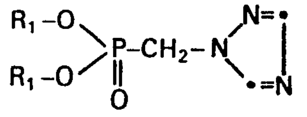

(III),

où les restes $R_1$ représentent indépendamment l'un de l'autre un phényle ou un alkyle $C_1$–$C_4$.

37. Sels de 1H-1,2,4-triazol-1-yl-méthylphosphonium de la formule générale IV,

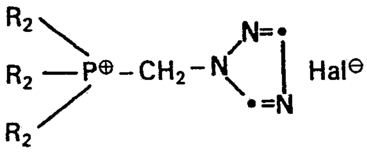 (IV),

où les restes $R_2$ représentent indépendamment l'un de l'autre un phényle ou un alkyle $C_1$–$C_4$ substitué éventuellement par un hydroxyle et Hal représente un chlore, un brome ou un iode.

38. Procédé de préparation des composés de la formule III, selon la revendication 36, caractérisé en ce qu'on fait réagir un 1-halogéno-méthyl--1H-1,2,4-triazol de la formule VI

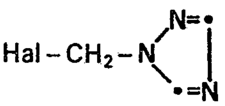 (VI),

où Hal représente un chlore, un brome ou un iode, soit avec un phosphite secondaire de la formule VII,

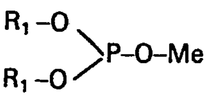 (VII),

où $R_1$ a la signification indiquée dans la revendication 36 et Me représente un atome de métal alcalin, ou avec un phosphite tertiaire de la formule VIII,

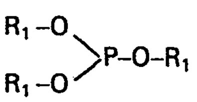 (VIII),

où $R_1$ a la signification indiquée dans la revendication 36.

39. Procédé de préparation des composés de la formule IV, selon la revendication 37, caractérisé en ce qu'on fait réagir un 1-halogéno-méthyl-1H-1,2,4-triazol de la formule VI avec une phosphine de la formule IX,

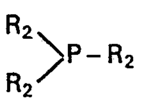 (IX),

où $R_2$ a la signification indiquée dans la revendication 37.

40. Procédé selon la revendication 38, caractérisé en ce qu'on fait réagir dans un solvant organique inerte le phosphite secondaire de la formule VII entre 50° et 150 °C ou le phosphite tertiaire de la formule VIII entre 100° et 180 °C avec l'halogénométhyltriazol de la formule VI.

41. Procédé selon la revendication 40, caractérisé en ce que la température réactionnelle pour le phosphite secondaire se situe entre 80° et 120 °C et pour le phosphite tertiaire entre 120° et 160 °C.

42. Procédé selon la revendication 39, caractérisé en ce que la réaction est effectuée dans un solvant organique inerte entre 30° et 120 °C.

43. Procédé selon la revendication 42, caractérisé en ce que la température réactionnelle se situe entre 50° et 100 °C.

## Claims

1. A process for the production of a 1-(2-phenylethyl)-1H-1,2,4-triazole of the general formula I

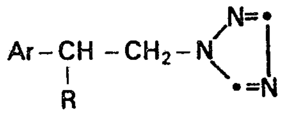 (I),

wherein Ar is phenyl or phenyl substituted by $C_1$–$C_4$alkyl, $C_1$–$C_4$alkoxy, cyano, nitro trifluoromethyl or by 1 to 3 halogen atoms, and R is hydrogen, $C_1$–$C_{10}$alkyl, $C_2$–$C_5$alkenyl, $C_3$–$C_8$cycloalkyl, $C_1$–$C_4$alkyl or $C_1$–$C_4$alkyl substituted by $C_3$–$C_8$cycloalkyl or by phenyl which may in turn be unsubstituted or substituted by halogen, $C_1$–$C_4$alkyl or $C_1$–$C_4$alkoxy, which process comprises reacting a phenylketone of the formula II

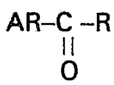 (II),

wherein Ar and R are as defined for formula I, first with a 1H-1,2,4-triazol-yl-methylphosphonate of the formula III

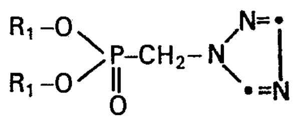 (III),

wherein each $R_1$ independently of the other is phenyl or $C_1$–$C_4$alkyl, or with a phosphonium salt of the formula IV

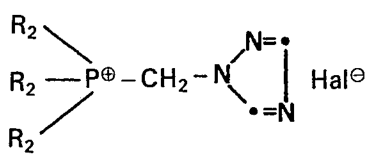 (IV),

wherein each of the radicals $R_2$ independently of one another is phenyl or $C_1$–$C_4$alkyl or $C_1$–$C_4$alkyl substituted by hydroxyl, and Hal is chlorine, bromine or iodine, in an inert solvent and in the presence of a strong base, to give a 1-(styryl-2)-1H-1,2,4-triazole of the formula V

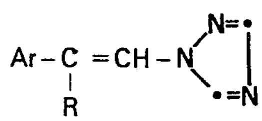 (V),

wherein Ar and R are as defined for formula I, and then converting this compound by catalytic hydrogenation into a compound of the formula I.

2. A process according to claim 1, wherein a phosphonate of the formula III is used for obtaining the 1-(styryl-2)-1H-1,2,4-triazole of the formula V.

3. A process according to claim 1, wherein a phosphonium salt of the formula IV is used for obtaining the 1-(styryl-2)-1H-1,2,4-triazole of the formula V.

4. A process according to claim 1, wherein the catalytic hydrogenation is carried out with a noble metal catalyst.

5. A process according to claim 4, wherein the catalyst is a noble metal oxide.

6. A process according to either of claims 4 or 5, wherein a mixture of catalysts is used.

7. A process according to claim 6, wherein a mixture of rhodium oxide and platinum oxide is used as catalyst.

8. A process according to any one of claims 1 to 3, wherein the catalytic hydrogenation is carried out in neutral to acid medium.

9. A process according to claim 8, wherein the acid medium is produced by addition of a mineral acid.

10. A process according to claim 8, wherein the hydrogenation is carried out in acetic acid or trifluoroacetic acid.

11. A process according to any one of claims 1 to 3, wherein the catalytic hydrogenation is carried out in the temperature range from 0° to 100 °C.

12. A process according to claim 11, wherein the temperature range is from 10° to 30 °C.

13. A process according to any one of claims 1 to 3, wherein the catalytic hydrogenation is carried out in the pressure range from 1 to 100 bar.

14. A process according to claim 13, wherein the pressure range is from 1 to 8 bar.

15. A process according to claim 2, wherein the 1H-1,2,4-triazolyl-methylphosphonate of the formula III is reacted first with a strong base and then with the phenylketone of the formula II.

16. A process according to claim 2, wherein the reaction of the phenyl ketone of the formula II with the 1H-1,2,4-triazolyl-methylphosphonate of the formula III is carried out by adding a solution of a mixture of phosphonate and phenylketone to a solution or suspension of a strong base.

17. A process according to either of claims 15 or 16, wherein the base is selected from the group consisting of organometallic compounds, alcoholates, metal hydrides or alkali amides.

18. A process according to claim 17, wherein the bases are metal hydrides or organometallic compounds.

19. A process according to either of claims 15 or 16 and either of claims 17 or 18, wherein an excess for base (up to twice the molar amount) is used.

20. A process according to either of claims 15 or 16, wherein the temperature range for the reaction of the phosphonate of the formula III with the base is from −40° to +40 °C and for the further reaction with the ketone of the formula II from −20° to +80 °C.

21. A process according to claim 20, wherein the temperature range in the first reaction step is from −20° to +40 °C and in the second from 0° to 60 °C.

22. A process according to either of claims 15 or 16, wherein the reaction is conducted in an inert, polar aprotic solvent.

23. A process according to claim 22, wherein the solvent is an ether, an acid amide or a sulfoxide.

24. A process according to claim 3, wherein the 1H-1,2,4-triazolyl-methylphosphonium salt of the formula IV is reacted first with a strong base and then with the phenyl ketone.

25. A process according to claim 24, wherein the base is selected from the group consisting of organometallic compounds, alcoholates, metal hydrides or alkali amides.

26. A process according to claim 25, wherein the base is a metal hydride or an organometallic compound.

27. A process according to claim 24 and either of claims 25 or 26, wherein an excess of base (up to twice the molar amount) is used.

28. A process according to claim 24, wherein the temperature range for the reaction of the phosphonium salt of the formula IV with the base is from −40° to +80 °C and for the further reaction with the ketone of the formula II from −20° to 110 °C.

29. A process according to claim 28, wherein the temperature range in the first reaction step is from −20° to +40 °C and in the second from 0° to 60 °C.

30. A process according to claim 24, wherein the reaction is conducted in an inert, polar aprotic solvent.

31. A process according to claim 30, wherein the solvent is an ether, an acid amide or a sulfoxide.

32. A process according to claim 2, wherein the methylphosphonate of the formula III is reacted first with an excess (up to twice the molar amount) of a base selected from the group consisting of organometallic compounds, alcoholates, metal hydrides or alkali amides, in an inert, polar aprotic solvent in the temperature range from −40° to +40 °C, and then with the phenyl ketone of the formula II in the temperature range from −20° to +80 °C to give the styryltriazole of the formula V, which is then hydrogenated with a noble metal catalyst in neutral or acid medium in the temperature range from 0° to 100 °C and in the pressure range from 1 to 100 bar.

33. A process according to claim 32, which comprises using a metal hydride or an organometallic compound as base for the reaction of the phosphonate and an ether, an acid amide or a sulfoxide as solvent, choosing a temperature in the range from −20° to +40 °C, carrying out the reaction with the ketone in the temperature range from 0° to 60 °C, and hydrogenating the styryltriazole with a mixture of rhodium oxide and platinum oxide at 10° to 30 °C and a pressure of 1 to 8 bar.

34. A process according to claim 3, wherein the methylphosphonium salt of the formula IV is

reacted first with an excess (up to twice the molar amount) of a base selected from the group consisting of the group of organometallic compounds, alcoholates, metal hydrides or alkali amides, in an inert, polar aprotic solvent in the temperature range from −40° to +80 °C and then with the phenylketone of the formula II in the temperature range from −20° to +110 °C to give the styryltriazole of the formula V, which is then hydrogenated with a noble metal catalyst in neutral to acid medium in the temperature range from 0° to 100 °C and in the pressure range from 1 to 100 bar.

35. A process according to claim 34, which comprises using a metal hydride or an organometallic compound as base for the reaction of the phosphonate and an ether, an acid amide or a sulfoxide as solvent, choosing the temperature in the range from −20° to +40 °C, carrying out the reaction with the ketone in the temperature range from 0° to 60 °C, and hydrogenating the styryltriazole with a mixture of rhodium oxide and platinum oxide at 10° to 30 °C and 1 to 8 bar.

36. A 1H-1,2,4-triazol-1-ylmethylphosphonate of the general formula III

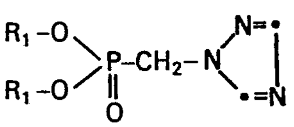

(III),

wherein each of the radicals $R_1$ independently of the other is phenyl or $C_1$–$C_4$alkyl.

37. A 1H-1,2,4-triazolyl-1-ylmethylphosphonium salt of the general formula IV

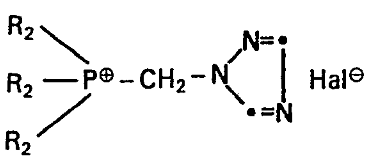

(IV),

wherein each of the radicals $R_2$ independently of one another is phenyl or $C_1$–$C_4$alkyl which is unsubstituted or substituted by hydroxyl, and Hal is chlorine, bromine or iodine.

38. A process for the production of a compound of the formula III according to claim 36, which process comprises reacting a 1-halomethyl-1H-1,2,4-triazole of the formula VI

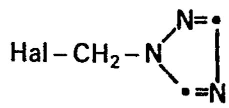

(VI),

wherein Hal is chlorine, bromine or iodine, either with a secondary phosphite of the formula VII

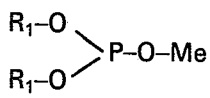

(VII),

wherein $R_1$ is as defined in claim 36 and Me is an alkali metal or with a tertiary phosphite of the formula VIII

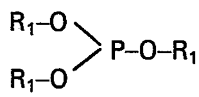

(VIII)

wherein $R_1$ is as defined in claim 36.

39. A process for the production of a compound of the formula IV according to claim 37, which process comprises reacting a 1-halomethyl-1H-1,2,4-triazole of the formula VI with a phosphine of the formula IX

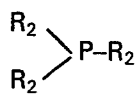

(IX),

wherein $R_2$ is as defined in claim 37.

40. A process according to claim 38, wherein the secondary phosphite of the formula VII is reacted in the temperature range from 50° to 150 °C, and the tertiary phosphite is reacted in the temperature range from 100° to 180 °C, with the halomethyltriazole of the formula VI, in an inert organic solvent.

41. A process according to claim 40, wherein the reaction temperature for the secondary phosphite is in the range from 80° to 120 °C, and for the tertiary phosphite in the range from 120° to 160 °C.

42. A process according to claim 39, wherein the reaction is carried out in an inert organic solvent in the temperature range from 30° to 120 °C.

43. A process according to claim 42, wherein the reaction temperature is in the range from 50° to 100 °C.